# EUROPEAN PATENT SPECIFICATION

(11) **EP 0 394 333 B1**
(45) Date of publication and mention of the grant of the patent: **15.03.1995**
(21) Application number: 89901102.7
(22) Date of filing: 21.12.1988
(51) Int. Cl.: A61K 31/70, A61K 31/725

(54) **USES OF SULPHATED SUGARS**
VERWENDUNG VON SULFATIERTEN ZUCKERN
UTILISATIONS DE SUCRES SULFATES

(30) Priority: 21.12.1987 DK 6740/87; 09.09.1988 DK 5054/88
(43) Date of publication of application: 31.10.1990
(60) Divisional application: 94202490.2
(73) Proprietor: BUKH MEDITEC A/S, DK-3500 Vaerloese (DK)
(72) Inventor: BAR-SHALOM, Daniel, DK-2980 Kokkedal (DK); BUKH, Niels, DK-2900 Hellerup (DK)
(74) Representative: Andersen, Henrik Rastrup
(86) International application number: DK8800217
(87) International publication number: WO8905646

(56) References cited:
- EP-A- 0 063 973
- EP-A- 0 097 625
- EP-A- 0 130 550
- EP-A- 0 136 100
- EP-A- 0 136 782
- EP-A- 0 230 023
- EP-A- 0 245 855
- EP-A- 0 254 845
- DE-A- 3 131 811
- US-A- 4 486 416
- US-A- 4 640 912
- American Journal of Gastroenterology, Vol. 80, No. 3, 1985, W.S. Brooks Jr., pp. 206-209

## Description

### FIELD OF INVENTION

The present invention relates to the use of sulphated saccharides for combatting or preventing aging of skin, including treating or preventing skin wrinkles.

### TECHNICAL BACKGROUND

Sulphated saccharides, primarily sucralfate, have previously been indicated for the treatment of gastric and duodenal ulcers (cf. US 3,432,489; EP 161816; EP 192640) and for the treatment of emesis and diarrhoea in dogs and cats (cf. EP 133880). In radio-labelled form, sucralfate has also been used as a diagnostic agent for the imaging of gastrointestinal mucosa, since the substance binds selectively to ulcerated areas in the stomach and upper small intestine (cf. EP 107209).

The American Journal of Castroenterology, 80(3), 1985, pp. 206-209; "Sucralfate: New Aspects in Therapy of Ulcers and Lesions" and the Second International Sucralfate Symposium Together With the World Congress of Castroenterology in Stockholm, suggest the use of sucralfate for a variety of non-ulcer applications, including the treatment of stomatitis, post-sclerotic ulcer, reflux oesophagitis and bile reflux oesophagitis as well as for counteracting the ulcerogenic effects of aspirin.

### SUMMARY OF THE INVENTION

It has surprisingly been found that polysulphated mono- or disaccharides exert very interesting effects when applied topically to the skin.

Thus, in one aspect, the present invention relates to the use of a sulphated mono- or disaccharide or a salt or complex thereof for combatting or preventing aging of skin, including treating or preventing skin wrinkles. In another aspect, the invention relates to the use of a mono- or disulphated saccharide or a salt or complex thereof for the manufacture of a composition for topical application for combatting or preventing aging of skin, including treating or preventing skin wrinkles.

In European Patent Application 230023, concerning the use of sulphated saccharides for the enhancement of wound healing, it is stated that sucralfate gives rise to inflammatory reactions when applied to a wound. It is also stated that low levels of 0.1-1.0 mg/ml of the polysulphated saccharide sucrose octasulphate in the form of its potassium salt, are preferred in order to avoid otherwise local haemorrhage or inflammation at the wound site.

The tolerance of an aqueous suspension of micronized sucralfate 2% has been tested in a rabbit eye study. There were no signs of any irritation or any kind of inflammatory reactions in conjunctiva, cornea or eye surroundings, and it was concluded that the test article was not an eye irritant (Example 9).

Extremely high tolerability is shown by sucralfate, which is the aluminium complex of sucrose octasulphate, as documented by the total absence of side-effects following its use in the treatment of peptic ulcer, and very high tolerability is shown by both sucralfate and sucrose octasulphate when used topically on the skin and mucosa.

It is furthermore contemplated that sulphated saccharides such as sucrose octasulphate stimulate tissue regenerative processes via other, not yet fully understood mechanisms.

It has been observed that one sulphated saccharide, sucralfate, when used internally in the treatment of peptic ulcers, binds preferentially to the surface of the ulcer. It is currently believed that this is a property common to sulphated saccharides, and that this binding is the result of an ability of sulphated saccharides to bind to proteoglycanes and hyaluronic acid. These structures are components of the surface of many cells, and they protect and stabilize the cell so the exterior cell surface remains intact. In other cases, e.g. in dermis and supportive tissue, proteoglycanes and hyaluronic acid form a protective matrix in which cells are embedded. Furthermore, it is known that certain sulphated saccharides, e.g. heparan sulphate, dextran sulphate and xylose sulphate, are hyaluronidase inhibitors.

Hyaluronidases are enzymes which catalytically cleave the glycosidic bonds of hyaluronic acid and glycosaminoglycanes. The decomposition of hyaluronic acid and glycosaminoglycanes by hyaluronidases therefore leads to exposure of the cells, via destruction of the cell surface or the supportive matrix substance, as well as to damage from various agents such as pathogens, inflammatory mediator substances, inflammatory agents and corrosive agents. Thus it is believed that by inhibiting hyaluronidases, sulphated saccharides promote the regeneration of the cell surface and the protective connective tissue matrix, and thereby effect an anti-inflammatory and tissue regenerative action.

Decomposition products of hyaluronic acid and glycosaminoglycanes may also act as mediator substances of inflammation themselves and via inhibition or modification of such decomposition, sucrose octasulphate and other sulphated saccharides may inhibit or modify inflammatory reactions and facilitate and modify tissue regeneration.

Thus it is contemplated that the above-mentioned pharmacological effects of sucrose octasulphate and other sulphated saccharides result in a "strengthening" of epithelial and mucosal linings. Apart from effecting an anti-inflammatory action, this strengthening of the exterior cell surface and connective tissue cell matrix will also make it more difficult for bacteria and virus to penetrate and colonize the cells and the tissue. Instead of a direct antimicrobial effect, an indirect effect will thus be obtained by applying sucrose octasulphate or other sulphated saccharides to mucosal and epithelial surfaces.

### DETAILED DISCLOSURE OF THE INVENTION

The sulphated saccharide used in accordance with the invention may be a monosaccharide, for instance xylose, fructose or glucose, an oligosaccharide, in particular a disaccharide such as sucrose, lactose, maltose or cellobiose.

In certain cases, it may be an advantage to use the sulphated saccharide in combination with a non-sulphated polysaccharide, for instance hyaluronic acid, *vide* Example 7.

The saccharide is preferably a polysulphated or persulphated saccharide, which means that two or more, possibly all, sulphur-containing moieties are present as substituents on the carbohydrate moiety.

In some cases, the sulphated saccharide may be complexed with or form a salt with a metal, e.g. an alkali or alkaline earth metal such as Na, K, Ca, Mg or Ba, or Al, Zn, Cu, Zr, Ti, Bi, Mn or Os, or with an organic base (e.g. an amino acid). The currently preferred salts are potassium and sodium salts.

Preferred oligosaccharides are mono- and disaccharides. Most preferably, the composition of the invention contains a persulphated disaccharide, optionally sucrose octasulphate.

The substance may, for instance, be prepared as disclosed in EP 230023.

Although there may be cases where the sulphated saccharide may be administered as such, it will typically be compounded with one or more cosmetically acceptable carriers or excipients to present it in a form which is suitable for topical application. In other words, it will be in the form of a liquid, semi-solid or solid topical or systemic preparation such as a powder, paste, ointment, lotion. gel, cream, salve, emulsion, solution, suspension, spray, sponge, strip, plaster, pad or dressing.

The preparation may be formulated in accordance with conventional cosmetical practice with cosmetical excipients conventionally used for topical applications such as pectin, gelatin and derivatives thereof, polylactic acid or polyglycolic acid polymers or copolymers thereof, cellulose derivatives such as methyl cellulose, carboxymethyl cellulose or oxidised cellulose, guar gum, acacia gum, karaya gum, tragacanth gum, bentonite, agar, carbomer, bladderwrack, ceratonia, dextran and derivatives thereof, ghatti gum, hectorite, ispaghula husk, polyvinylpyrrolidone, silica and derivatives thereof, xanthan gum, kaolin, talc, starch and derivatives thereof, paraffin, water, vegetable and animal oils, polyethylene, polyethylene oxide, polyethylene glycol, polypropylene glycol, glycerol, ethanol, propanol, propylene glycol, (glycols, alcohols), fixed oils, sodium, potassium, aluminium, magnesium or calcium salts (such as the chloride, carbonate, bicarbonate, citrate, gluconate, lactate, acetate, gluceptate or tartrate).

The preparation may also contain other additives such as emulsifiers, stabilizing agents, preservatives, etc.

Plasters, sponges, strips, pads or other dressings may be prepared by impregnating a dressing material such as cotton wool or gauze or a polymeric substance with a solution or suspension of the sulphated saccharide followed by drying. Alternatively, a paste, lotion, cream or gel containing the sulphated saccharide may be spread over the dressing material, conveniently immediately prior to use.

The preparation generally comprises the sulphated saccharide in an amount of 0.001-99%, typically 0.01-75%, more typically 0.1-20%, especially 1-10% by weight of the total preparation. In particular, when the sulphated saccharide is sucrose octasulphate, a preferred concentration thereof in the preparation is often 0.5-50%, especially 0.5-25%, such as 1-10%. It is suitably applied 1-10 times a day, dependent on the type and severity of the condition to be treated.

The concentration of the sulphated saccharide to be used in each particular case will of course depend upon the type of preparation and the intended use, but also on the solubility characteristics of the sulphated saccharide and, for sparingly soluble and substantially insoluble sulphated saccharides, on the particle size thereof; the smaller the particle size, the faster will be the distribution of even sparingly soluble or even substantially insoluble sulphated saccharides or complexes thereof. Insoluble or sparingly soluble salts or complexes of sulphated saccharides are preferably used in the form of a fine powder, for example having a particle size of 200 »m or less, such as 100 »m or less. Examples of very small particle sizes which may be desirable for certain purposes are e.g. 50 »m or less, such as 20 »m or less, in certain cases 10 »m or less, such as 5 »m or less.

The preparation may contain other active agents than the sulphated saccharide, such as sun protective agents, vitamins and vitamin derivatives or analogues.

Particular conditions for which use of the sulphated saccharide is indicated include:
Cosmetically against wrinkles and aging skin, both as active and prophylactic treatment.

### EXAMPLE 1

A topical powder preparation was prepared from the following ingredients:

| | |
|---|---|
| Sucralfate* | 30 g |
| Pectin | 10 g |
| Gelatin | 10 g |
| Carboxymethylcellulose | 10 g |

| | |
|---|---|
| * Provided by Abic Laboratories, Israel, in finely divided form. | |

The finely divided sucralfate (particle size 2-100 »m) was thoroughly mixed with the other ingredients in finely divided form (particle size <250 »m) to produce a powder.

### EXAMPLE 2

A topical ointment preparation was prepared from the following ingredients:

| | |
|---|---|
| Sucralfate | 30 g |
| Pectin | 10 g |
| Gelatin | 10 g |
| Carboxymethylcellulose | 10 g |
| Fractionated coconut oil | 60 g |

The finely divided sucralfate (particle size 2-100 »m) was thoroughly mixed with the other ingredients in finely divided form. The fractionated coconut oil was added to the resulting powder to a suitable consistency and a substantially homogeneous dispersion of the particulate components.

### EXAMPLE 3

A topical ointment preparation was prepared from the following ingredients:

| | |
|---|---|
| Sucralfate | 30 g |
| Hyaluronic acid | 0.6 g |
| Pectin | 10 g |
| Gelatin | 10 g |
| CMC | 10 g |
| Fractionated coconut oil | 60 g |

The finely divided sucralfate (particle size 2-100 »m) was thoroughly mixed with the other ingredients in finely divided form. The fractionated coconut oil was added to the resulting powder to a suitable consistency and a substantially homogeneous dispersion of the particulate components.

### EXAMPLE 4 (not according to the invention)

A topical eye preparation was prepared from the following ingredients:

| | |
|---|---|
| Sucralfate * | 2 % |
| Carbopol 934 | 0,5 % |
| Mannitol | 5 % |
| Benzalkoniumchloride | 0,01 % |
| Sodium EDTA | 0,05 % |
| Sodium hydroxide q.s. | ad pH 6 |
| Sterile water | ad 100 % |

| | |
|---|---|
| * Micronized sucralfate (particle size 10 »m), provided by Guilini Chemie, W. Germany. | |

### EXAMPLE 5

### Eye preparation (not according to the invention)

An eye preparation was prepared from the following ingredients:

| | |
|---|---|
| Sucralfate* | 2% |
| Propyl methyl cellulose | 0.35% |
| Benzalkonium chloride | 0.01% |
| Sodium EDTA | 0.05% |
| Sodium chloride | 0.8% |
| Sterile water q.s. | |

| | |
|---|---|
| * Micronized (10 »m), provided by Guilini Chemie Ludwigshafen, W. Germany. | |

### EXAMPLE 6

A topical preparation for skin was prepared by mixing 5% by weight of a sucralfate powder (particle size 50-100 »m. provided by Guilini Chemie, W. Germany) with a mixture of cetanole, adeps lanae purificatae, isopropyl myristas, Tween 60, Span 60, dimeticone, glycerol, sorbic acid and sterile water.

### EXAMPLE 7

A) A topical preparation for skin was prepared from the following ingredients:

| | |
|---|---|
| Sucralfate powder * | 5 % |
| Paraffin oils, glycerine, cetyl alcohol | 55 % |
| Quarternary ammonium compounds | 0,7 % |
| Stearyl alcohol | 3 % |
| Eucalyptus oil q.a. | |

| | |
|---|---|
| * Micronized sucralfate (≦10 »m), provided by Guilini Chemie, W. Germany. | |

### EXAMPLE 8

### Human Clinical Trials

A) (not according to the invention) Four females aged 48-71 with vulvovaginitis symptoms were freed of their pruritus after topical application of sucralfate ointment twice daily for four months. The preparation comprised 5% by weight of sucralfate powder mixed in a fatty vehicle containing herbal extracts of chamomile (6%) and arnica (4%). The ointment was applied morning and evening. No side effects have been seen during treatment with sucralfate ointment during this period.
B) The sucralfate ointment described in Example 8 A) was tested on facial wrinkles around the eyes. Three females aged 38-45 have used the ointment twice daily, and a beneficial effect has been reported after 1-2 weeks of treatment.

### EXAMPLE 9

### Rabbit eye tolerance test of sucralfate eye drops

The primary eye irritative effect of the sucralfate eye drops of Example 5 was tested in rabbits. The testing was done on four SPF albino female rabbits. Only the left eye was treated and the right eye served as an untreated control. About 0.1 ml of the test preparation was applied to the eye by gently pulling the lower eyelid away from the eyeball to form a cup into which the test substance was placed. The lids were then gently held together for about one second. The eyes were examined and the grade of occular reaction was recorded 1 hour later. 24 hours later an examination was performed before and after installation of ocoluguttae fluoresceini. After the examination the eyes were rinsed with 20 ml of a 0.9% sodium chloride solution. The eyes were also examined 48 and 72 hours after treatment. Cornea, iris and conjunctiva (including discharge) were inspected, and any reactions and changes were observed and scored. Slight discharge of conjunctiva was observed in two of the rabbits at the first examination. No reactions of conjunctiva, iris, or cornea were observed in any of the rabbits at the 24, 48 and 72 hour examinations.

Mean score values, as determined by a variety of different standard criteria, for cornea opacity, iris lesion, redness of conjunctiva and oedema of conjunctiva (chemosis) were all 0.0.

According to the criteria in the Official Journal of the European Communities, L 257, 1983, the directive of the commission, 83/467/EEC of July 29, 1983, and the above mean values, it must be concluded that the tested sucralfate in a 2% aqueous suspension shall not be classified on eye irritant.

## Claims

1. Use of a sulphated mono- or disaccharide or a salt or complex thereof for combatting or preventing aging of skin, including treating or preventing skin wrinkles.

2. Use according to claim 1, wherein the saccharide is a monosaccharide selected from the group consisting of xylose, fructose and glucose.

3. Use according to claim 1, wherein the saccharide is a disaccharide selected from the group consisting of sucrose, lactose, maltose and cellobiose.

4. Use according to claim 1, wherein the sulphated mono- or disaccharide is combined with a non-sulphated polysaccharide, for instance hyaluronic acid.

5. Use according to any of claims 1-4, wherein the mono- or disaccharide is a polysulphated mono- or disaccharide, in particular a persulphated mono- or disaccharide.

6. Use according to claim 5, wherein the polysulphated saccharide is sucrose octasulfate.

7. Use according to claim 1, wherein the substance with which the sulphated mono- or disaccharide is complexed or with which it forms a salt is a metal, e.g. an alkali or alkaline earth metal (e.g. Na, K, Ca, Mg or Ba) or Al, Zn, Cu, Zr, Ti, Bi, Mn or Os, or is an organic base (e.g. an amino acid).

8. Use according to claim 6, wherein the polysulphated saccharide is the potassium or sodium salt of sucrose octasulphate.

9. Use according to claim 6 wherein the polysulphated saccharide is the aluminium complex of sucrose octasulphate, sucralfate.

10. Use of a sulphated mono- or disaccharide or a salt or complex thereof for the manufacture of a composition for topical application for combatting or preventing aging of skin, including treating or preventing skin wrinkles.

11. Use according to claim 10, wherein the saccharide is a monosaccharide selected from the group consisting of xylose, fructose and glucose.

12. Use according to claim 10, wherein the saccharide is a disaccharide selected from the group consisting of sucrose, lactose, maltose and cellobiose.

13. Use according to claim 10, wherein the sulphated mono- or disaccharide is combined with a non-sulphated polysaccharide, for instance hyaluronic acid.

14. Use according to any of claims 10-13, wherein the mono- or disaccharide is a polysulphated mono- or disaccharide, in particular a persulphated mono- or disaccharide.

15. Use according to claim 14, wherein the polysulphated saccharide is sucrose octasulfate.

16. Use according to claim 10, wherein the substance with which the sulphated mono- or disaccharide is complexed or with which it forms a salt is a metal, e.g. an alkali or alkaline earth metal (e.g. Na, K, Ca, Mg or Ba) or Al, Zn, Cu, Zr, Ti, Bi, Mn or Os, or is an organic base (e.g. an amino acid).

17. Use according to claim 15 wherein the polysulphated saccharide is the potassium or sodium salt of sucrose octasulphate.

18. Use according to claim 15, wherein the polysulphated saccharide is the aluminium complex of sucrose octasulphate, sucralfate.

19. Use according to any of claims 10-18, wherein the composition is in a form suitable for topical application to the skin, e.g. a powder, paste, ointment, lotion, gel, cream, salve, emulsion, solution, suspension, spray, sponge, strip, plaster, pad or dressing.

20. Use according to any of claims 10-19, wherein the composition is in the form of a topical preparation comprising the sulphated mono- or disaccharide in an amount of 0.001-99%, typically 0.01-75%, more typically 0.1-20%, especially 1-10%, by weight of the composition.

## Patentansprüche

1. Verwendung eines sulfatierten Mono- oder Disaccharids oder eines Salzes oder Komplexes davon zum Bekämpfen oder Verhindern des Alterns der Haut, einschließlich der Behandlung oder Verhinderung von Falten.

2. Verwendung nach Anspruch 1, worin das Saccharid ein Monosaccharid, ausgewählt aus der Gruppe bestehend aus Xylose, Fructose und Glucose, ist.

3. Verwendung nach Anspruch 1, worin das Saccharid ein Disaccharid, ausgewählt aus der Gruppe bestehend aus Sucrose, Lactose, Maltose und Cellobiose, ist.

4. Verwendung nach Anspruch 1, worin das sulfatierte Mono- oder Disaccharid mit einem nicht sulfatierten Polysaccharid, z.B. Hyaluronsäure, kombiniert ist.

5. Verwendung nach einem der Ansprüche 1-4, worin das Mono- oder Disaccharid ein polysulfatiertes Mono- oder Disaccharid, insbesondere ein persulfatiertes Mono- oder Disaccharid ist.

6. Verwendung nach Anspruch 5, worin das polysulfatierte Saccharid Sucroseoctasulfat ist.

7. Verwendung nach Anspruch 1, worin die Substanz, mit der das sulfatierte Mono- oder Disaccharid komplexiert ist oder mit der es ein Salz bildet, ein Metall, z.B. ein Alkali- oder Frdalkalimetall (z.B. Na, K, Ca, Mg oder Ba) oder Al, Zn, Cu, Zr, Ti, Bi, Mn oder Os, oder eine organische Base (z.B. eine Aminosäure) ist.

8. Verwendung nach Anspruch 6, worin das polysulfatierte Saccharid das Kalium- oder Natriumsalz von Sucroseoctasulfat ist.

9. Verwendung nach Anspruch 6, worin das polysulfatierte Saccharid der Aluminiumkomplex von Sucroseoctasulfat, Sucralfat ist.

10. Verwendung eines sulfatierten Mono- oder Disaccharids oder eines Salzes oder Komplexes davon zur Herstellung einer Zusammensetzung zur topischen Anwendung zum Bekämpfen oder Verhindern des Alterns der Haut, einschließlich der Behandlung oder Verhinderung von Falten.

11. Verwendung nach Anspruch 10, worin das Saccharid ein Monosaccharid, ausgewählt aus der Gruppe bestehend aus Xylose, Fructose und Glucose, ist.

12. Verwendung nach Anspruch 10, worin das Saccharid ein Disaccharid, ausgewählt aus der Gruppe bestehend aus Sucrose, Lactose, Maltose und Cellobiose, ist.

13. Verwendung nach Anspruch 10, worin das sulfatierte Mono- oder Disaccharid mit einem nicht sulfatierten Polysaccharid, z.B. Hyaluronsäure, kombiniert ist.

14. Verwendung nach einem der Ansprüche 10-13, worin das Mono- oder Disaccharid ein polysulfatiertes Mono- oder Disaccharid, insbesondere ein persulfatiertes Mono- oder Disaccharid ist.

15. Verwendung nach Anspruch 14, worin das polysulfatierte Saccharid Sucroseoctasulfat ist.

16. Verwendung nach Anspruch 10, worin die Substanz, mit der das sulfatierte Mono- oder Disaccharid komplexiert ist oder mit der es ein Salz bildet, ein Metall, z.B. ein Alkali- oder Erdalkalimetall (z.B. Na, K, Ca, Mg oder Ba) oder Al, Zn, Cu, Zr, Ti, Bi, Mn oder Os, oder eine organische Base (z.B. eine Aminosäure) ist.

17. Verwendung nach Anspruch 15, worin das polysulfatierte Saccharid das Kalium- oder Natriumsalz von Sucroseoctasulfat ist.

18. Verwendung nach Anspruch 15, worin das polysulfatierte Saccharid der Aluminiumkomplex von Sucroseoctasulfat, Sucralfat ist.

19. Verwendung nach einem der Ansprüche 10-18, worin die Zusammensetzung in einer für die topische Anwendung auf die Haut geeigneten Form, z.B. als Pulver, Paste, Salbe, Lotion, Gel, Creme, Balsam, Emulsion, Lösung, Suspension, Spray, Schwamm, Streifen, Pflaster, Kissen oder Verband, vorliegt.

20. Verwendung nach einem der Ansprüche 10-19, worin die Zusammensetzung in Form eines topischen Präparats vorliegt, welches das sulfatierte Mono- oder Disaccharid in einer Menge von 0,001-99%, typischerweise 0,01-75%, noch typischer 0,1-20%, insbesondere 1-10%, bezogen auf das Gewicht der Zusammensetzung, umfaßt.

## Revendications

1. Utilisation d'un mono- ou di-saccharide sulfaté ou d'un sel ou complexe de celui-ci pour combattre ou empêcher le vieillissement de la peau et, notamment, pour traiter ou empêcher la formation de rides sur la peau.

2. Utilisation selon la revendication 1, dans laquelle le saccharide est un monosaccharide choisi parmi le xylose, le fructose et le glucose.

3. Utilisation selon la revendication 1, dans laquelle le saccharide est un disaccharide choisi parmi le saccharose, le lactose, le maltose et le cellobiose.

4. Utilisation selon la revendication 1, dans laquelle le mono- ou di-saccharide sulfaté est combiné avec un polysaccharide non sulfaté, par exemple l'acide hyaluronique.

5. Utilisation selon l'une quelconque des revendications 1 à 4, dans laquelle le mono- ou disaccharide est un mono- ou disaccharide polysulfaté, en particulieur un mono-ou disaccharide persulfaté.

6. Utilisation selon la revendication 5, dans laquelle le saccharide polysulfaté est un octasulfate de saccharose.

7. Utilisation selon la revendication 1, dans laquelle la substance avec laquelle le mono- ou disaccharide sulfaté est complexé ou avec laquelle il forme un sel est un métal , par exemple un métal alcalin ou alcalinoterreux (par exemple de Na, K, Ca, Mg ou Ba) ou Al, Zn, Cu, Zr, Ti, Bi, Mn ou Os, ou est une base organique (par Exemple un amino-acide).

8. Utilisation selon la revendication 6, dans laquelle le saccharide polysulfaté est le sel de potassium ou de sodium de l'octasulfate de saccharose.

9. Utilisation selon la revendication 6, dans laquelle le saccharide polysulfaté est le complexe d'aluminium d'octasulfate de saccharose, à savoir un sucralfate.

10. Utilisation d'un mono- ou di-saccharide sulfaté ou d'un sel ou complexe de celui-ci pour la fabrication d'une composition pour application locale destinée à combattre ou à empêcher le vieillissement cutané, notamment traiter ou empêcher les rides.

11. Utilisation selon la revendication 10, dans laquelle le saccharide est un monosaccharide choisi parmi le xylose, le fructose et le glucose.

12. Utilisation selon la revendication 10, dans laquelle le saccharide est un disaccharide choisi parmi le saccharose, le lactose, le maltose et le cellobiose.

13. Utilisation selon la revendication 10, dans laquelle le mono ou di-saccharide sulfaté est combiné avec un polysccharide non sulfaté par exemple l'acide hyaluronique.

14. Utilisation selon l'une quelconque des revendications 10 à 13, dans laquelle le mono- ou di-saccharide est un mono- ou disaccharide polysulfatée, en particulier un mono- ou disaccharide persulfaté.

15. Utilisation selon la revendication 14, dans laquelle le saccharide polysulfaté est l'octasulfate de saccharose.

16. Utilisation selon la revendication 10, dans laquelle la substance avec laquelle le mono- ou di-saccharide sulfaté est complexée ou avec laquelle il forme un sel est un métal, par example, un métal alcalin ou alcalino-terreux (par exemple Na, K, Ca, Mg ou Ba) ou Al, Zn, Cu, Zr, Ti, Bi, Mn ou Os, ou est une base organique, (par exemple un amino-acide).

17. Utilisation selon la revendication 15, dans laquelle le saccharide polysulfaté est le sel de potassium ou de sodium de l'octasulfate de saccharose.

18. Utilisation selon la revendication 15, dans laquelle le saccharide polysulfaté est le complexe d'aluminium de l'octasulfate de saccharose, à savoir un sucralfate.

19. Utilisation selon l'une quelconque des revendications 10 à 18, dans laquelle la composition est sous une forme appropriée pour les applications locales à la peau, par exemple une poudre, une pâte, un onguent, une lotion, un gel, une crème, un baume, une émulsion, une solution, une suspension, une pulvérisation, une éponge, un ruban, un plâtre, un tampon ou un pansement.

20. Utilisation selon l'une quelconque des revendications 10 à 19, dans laquelle la composition est sous forme d'une préparation à usage locale comprenant le mono- ou disaccharide sulfaté en une quantité de 0,001 à 99%, normalement de 0,01 à 75%, plus normalement de 0,1 à 20% et, surtout, de 1 à 10% en poids de la composition.
